# EUROPEAN PATENT APPLICATION

(11) **EP 2 647 344 A1**
(43) Date of publication of application: **09.10.2013**
(21) Application number: 13162546.9
(22) Date of filing: 05.04.2013
(51) Int. Cl.: A61B 17/34, A61B 8/08

(54) **Acoustic probe and acoustic diagnostic system including the same**

(30) Priority: 06.04.2012 KR 20120036244
(71) Applicant: Samsung Medison Co., Ltd., Gangwon-do 250-870 (KR)
(72) Inventor: Hyun, Dong-gyu, 250-870 Gangwon-do (KR); Kim, Seung-tae, 250-870 Gangwon-do (KR); Song, Mi-ran, 250-870 Gangwon-do (KR); Ahn, Mi-jeoung, 250-870 Gangwon-do (KR); Lee, Suk-jin, 250-870 Gangwon-do (KR); Lee, Jun-kyo, 250-870 Gangwon-do (KR)
(74) Representative: Schmid, Wolfgang

(57) **Abstract**

An acoustic probe and an acoustic diagnostic system. The acoustic probe includes a housing; a transducer, which is arranged inside the housing, transmits an acoustic wave to a target object, and receives an acoustic echo signal from the target object; and a magnetic field generator, which is arranged inside the housing and generates a magnetic field to the target object.

## Description

### CROSS-REFERENCE TO RELATED PATENT APPLICATION

This application claims the benefit of Korean Patent Application No. 10-2012-0036244, filed on April 6, 2012, in the Korean Intellectual Property Office, the disclosure of which is incorporated herein in its entirety by reference.

### BACKGROUND OF THE INVENTION

### 1. Field of the Invention

The present invention relates to an acoustic probe including a magnetic field generator and an acoustic diagnostic system including the same.

### 2. Description of the Related Art

An acoustic diagnostic system transmits acoustic signals from a surface of a target object toward a predetermined location inside the target object and obtains images regarding tomography of a soft-tissue or blood flow by using information regarding acoustic signals reflected by tissues inside the target object.

Such an acoustic diagnostic system is small, is inexpensive, and is capable of displaying images in real time. Furthermore, an acoustic diagnostic system is highly safe without exposure to X-ray. Therefore, an acoustic diagnostic system is widely used with other imaging diagnostic devices, such as an X-ray diagnostic device, a computerized tomography (CT) scanner, a magnetic resonance imaging (MRI) device, and a nuclear medicine diagnostic device.

Generally, acoustic biopsy is widely performed to diagnose a tumor. The biopsy refers to cutting a small piece of tissue from a lesion of a patient and observing the cut tissue with the naked eyes or a microscope. During a biopsy, a performer may fail to figure out exact path of a needle and exact location of the tip of the needle, and thus a medical malpractice may occur.

To prevent a medical malpractice during a biopsy, techniques for showing the tip of a needle and path of the needle are being developed. Meanwhile, a magnetic field generator for generating a magnetic field is arranged separately from an acoustic diagnostic device in the related art. Since a magnetic field generator is arranged separately from an acoustic diagnostic device, it is difficult to figure out the exact location of a needle.

### SUMMARY OF THE INVENTION

The present invention provides an acoustic probe capable of easily figuring out relative locations between a needle and the acoustic probe and an acoustic diagnostic system including the same.

According to an aspect of the present invention, there is provided an acoustic probe including a housing; a transducer, which is arranged inside the housing, transmits an acoustic wave to a target object, and receives an acoustic echo signal from the target object; and a magnetic field generator, which is arranged inside the housing and generates a magnetic field to the target object.

A space in which the magnetic field is formed includes a direction in which an acoustic wave propagates.

A direction of the magnetic field generated by the magnetic field generator is identical to a direction in which an acoustic wave generated by the transducer propagates.

The magnetic field generator is formed of a coil.

Weight of the acoustic probe is less than or equal to about 3Kg.

The acoustic probe further includes a needle to be inserted into the target object; and a location sensor, which is arranged at the needle for detecting location of the needle.

The location sensor detects a relative location of location sensor with respect to the magnetic field generator.

The acoustic probe further includes a location calculating unit, which calculates a relative location of the location sensor with respect to the magnetic field generator based on signals output by the magnetic field generator and/or the location sensor and calculates a relative location of the needle with respect to the magnetic field generator based on the relative location of the location sensor with respect to the magnetic field generator.

The location calculating unit is arranged in the housing or a connector that is connected to an acoustic diagnostic system.

The acoustic probe further includes a signal line, which interconnects the needle and the housing and transmits a signal detected by the location sensor to the location calculating unit.

The signal line is detachable from the housing.

The acoustic probe further includes a magnetic field control unit, which controls magnetic field generation of the magnetic field generator.

The magnetic field control unit is arranged in the housing or the connector that is connected to the acoustic diagnostic system.

Location of the magnetic field generator is a relative location of the magnetic field generator with respect to the location sensor.

According to another aspect of the present invention, there is provided an acoustic diagnostic system including the acoustic probe of claim 1; and an acoustic signal processing unit, which generates an acoustic image by using the acoustic echo signals transmitted by the acoustic probe.

The acoustic diagnostic system further includes a display unit, which displays a needle icon corresponding to the needle and the acoustic image.

Relative location of the needle with respect to the acoustic probe is displayed by a position of the needle icon.

### BRIEF DESCRIPTION OF THE DRAWINGS

The above and other features and advantages of the present invention will become more apparent by describing in detail exemplary embodiments thereof with reference to the attached drawings in which:
FIG. 1 is a front view of an acoustic diagnostic system including an acoustic probe, according to an embodiment of the present invention;
FIG. 2 is a block diagram of the acoustic diagnostic system; and
FIG. 3 is a respective view of the acoustic probe to which a needle is connected.

### DETAILED DESCRIPTION OF THE INVENTION

Although the terms used in the present invention are selected from generally known and used terms, some of the terms mentioned in the description of the present invention have been selected by the applicant at his or her discretion, the detailed meanings of which are described in relevant parts of the description herein. Furthermore, it is required that the present invention is understood, not simply by the actual terms used but by the meaning of each term lying within.

The terminology used herein is for the purpose of describing particular embodiments only and is not intended to be limiting of the invention. As used herein, the singular forms "a", "an" and "the" are intended to include the plural forms as well, unless the context clearly indicates otherwise. It will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof.

The terminology "acoustic image" refers to an image regarding a target object obtained by using acoustic waves throughout. The terminology "target object" may refer to a part of a human body. For example, examples of the target body may include organs, such as a liver, a heart, and a uterus, or a fetus.

The terminology "user" may be a medical expert, such as a doctor, a nurse, a medical technologist, a and medical image expert, but is not limited thereto.

The present invention will now be described more fully with reference to the accompanying drawings, in which exemplary embodiments of the invention are shown. The invention may, however, be embodied in many different forms and should not be construed as being limited to the embodiments set forth herein; rather, these embodiments are provided so that this disclosure will be thorough and complete, and will fully convey the concept of the invention to those skilled in the art. In the description of the present invention, certain detailed explanations of related art are omitted when it is deemed that they may unnecessarily obscure the essence of the invention. Like reference numerals in the drawings denote like elements, and thus their description will be omitted.

As used herein, the term "and/or" includes any and all combinations of one or more of the associated listed items.

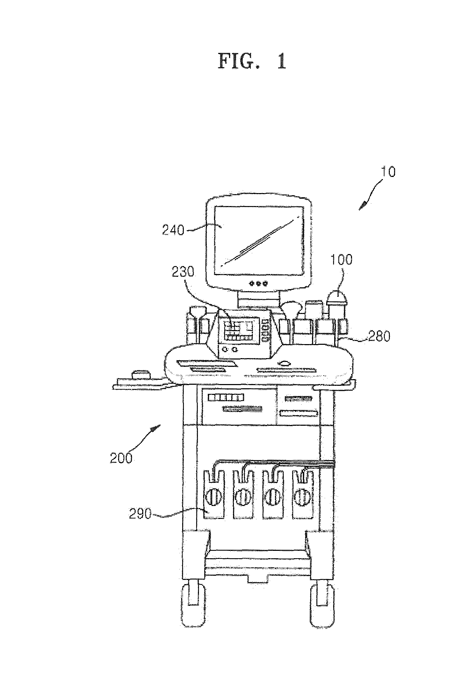

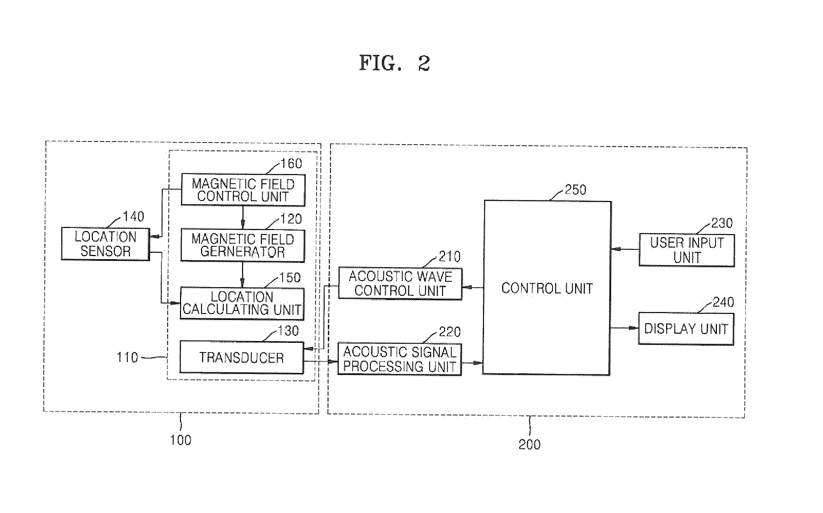

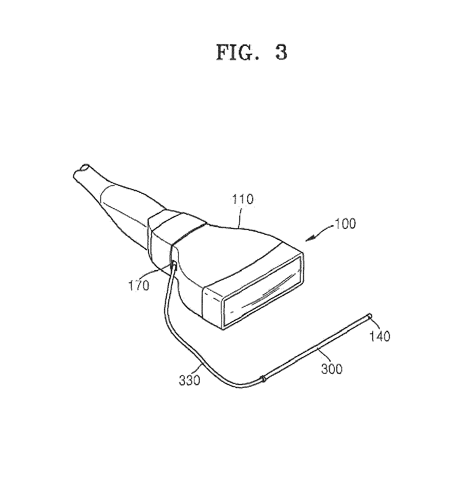

FIG. 1 is a front view of an acoustic diagnostic system 10 including an acoustic probe 100, according to an embodiment of the present invention, and FIG. 2 is a block diagram of the acoustic diagnostic system 10. FIG. 3 is a respective view of the acoustic probe 100 to which a needle 300 is connected.

The acoustic probe 100 according to the present invention may be used not only in the acoustic diagnostic system 10, but also in any of various acoustic probe-related devices. For convenience of explanation, a case in which the acoustic probe 100 according to the present invention is used in an acoustic diagnostic device will be described below.

First, referring to FIG. 1, the acoustic diagnostic system 10 according to the present invention includes the acoustic probe 100, which transmits acoustic waves to a target object and receives acoustic echo signals from the target object, and a main body 200, which includes a user input device 230, such as operating buttons, and a display unit 240 and generates acoustic images of the target object. A cable 280 of the acoustic probe 100 may be connected to the main body 200 via a first connector 290.

First, the acoustic probe 100 may include a magnetic field generator 120, which is arranged inside a housing 110 and generates a magnetic field with respect to a target object, and a transducer 130, which is arranged inside the housing 110, transmits acoustic waves to the target object, and receives acoustic echo signals from the target object.

The magnetic field generator 120 generates a magnetic field according to a voltage. A space in which the magnetic field is formed may include a direction in which an acoustic wave propagates. The magnetic field generator 120 may be formed of a coil. A direction of the magnetic field generated by the magnetic field generator 120 may be identical to a direction in which an acoustic wave generated by the transducer 130 propagates. For example, if the magnetic field generator 120 is formed of a solenoid, a direction of the solenoid may be identical to the direction in which an acoustic wave propagates. Alternatively, the magnetic field generator 120 may be formed of three axial coils that are perpendicular to one another, where direction of a magnetic field generated by one of the three axial coils may be identical to the direction in which an acoustic wave generated by the transducer 130 propagates. In a case where the magnetic field generator 120 is formed of three axial coils, the coils may sequentially generate magnetic fields as time passes.

The transducer 130 according to an embodiment of the present invention may be at least one from among a one-dimensional (1 D) transducer, a two-dimensional (2D) transducer, a three-dimensional (3D) transducer, and a four-dimensional (4D) transducer. Therefore, the acoustic diagnostic system 10 may generate acoustic images regarding a target object based on acoustic echo signals received from the target object.

Furthermore, the needle 300 that may be inserted into a target object may be connected to the acoustic probe 100, and the acoustic probe 100 may further include a location sensor 140 to detect location of the needle 300. The location sensor 140 may be arranged at the tip of the needle 300. For example, the location sensor 140 may detect a magnetic field by inducing change of the magnetic field. The needle 300 is connected to the housing 110 of the acoustic probe 100 via a signal line 330 and a second connector 170, and thus a result detected by the location sensor 140 may be applied to a location calculating unit 150.

The location sensor 140 may detect magnetic field signals generated by the magnetic field generator 120. The location sensor 140 may be formed of a coil. The location sensor 140 may be attached to the needle 300. For example, if the location sensor 140 is formed of a solenoid, a direction of the solenoid may be identical to the lengthwise direction of the needle 300. The location sensor 140 may be arranged at the tip of the needle 300. Alternatively, the location sensor 140 may also be formed of three axial coils that are perpendicular to one another, where axial direction of one of the three axial coils may be identical to the lengthwise direction of the needle 300. The location sensor 140 may also have any of various configurations other than the configuration for detecting magnetic field signals.

FIG. 3 is a respective view of the acoustic probe 100 to which the needle 300 is connected. As shown in FIG. 3, the acoustic probe 100 may include a second connector 170 that is arranged at the housing 110 to which the signal line 330 extending from the needle 300 is connected. Therefore, the signal line 330 may apply a signal detected by the location sensor 140 to a location calculating unit 150 described below. The signal line 330 may be attached to and detached from the second connector 170.

The acoustic probe 100 may further include a magnetic field control unit 160 for controlling generation of a magnetic field. In a case where the location sensor 140 detects a magnetic field by inducing change of the magnetic field, the magnetic field control unit may also control the location sensor 140.

Furthermore, the acoustic probe 100 may further include a location calculating unit 150 which calculates a relative location relationship between the location sensor 140 and the magnetic field generator 120 based on a signal output by at least one from between the location sensor 140 and the magnetic field generator 120.

The location sensor 140 is arranged at the tip of the needle 300 and the magnetic field generator 120 is arranged inside the acoustic probe 100. Therefore, the location calculating unit 150 may calculate a relative location relationship of the location sensor 140 with respect to the magnetic field generator 120 based on a relative location relationship of the magnetic field generator 120 with respect to the location sensor 140. A method of calculating a location based on a magnetic field known in the art may be employed in the present embodiment, and thus detailed description thereof will be omitted.

Generally, in calculation of a location based on a magnetic field, either the magnetic field generator 120 or the location sensor 140 is fixed, and a location is calculated based on intensity of a magnetic field due to movement of the other not fixed. However, the acoustic probe 100 generally moves while contacting surface of a target object. Therefore, if a location of the needle 300 is calculated based on a result detected by the location sensor 140 arranged at the top of the needle 300 while the magnetic field generator 120 is fixed to a location other than the acoustic probe 100, it is difficult to figure out a relative location relationship between an acoustic image generated by using the acoustic probe 100 and the needle 300. Furthermore, to figure out a relative location relationship between an acoustic image and the needle 300, it is necessary to arrange an additional location sensor at the acoustic probe 100, where it becomes complicated to calculate a location by using the plurality of location sensors.

However, according to the present invention, the magnetic field generator 120 is arranged inside the acoustic probe 100, and thus a relative location relationship between a magnetic field and the needle 300 may be easily figured out based on a relative location relationship between the magnetic field generator 120 and the location sensor 140.

The acoustic probe 100 described above may weigh less than or equal to about 3Kg. The acoustic probe 100 may preferably weight less than or equal to about 2Kg or about 1Kg. Even if the acoustic probe 100 includes some of the components of the main body 200, the acoustic probe 100 may weigh less than or equal to about 3Kg. If the acoustic probe 100 weighs too much, it may be inconvenient for a user to use the acoustic probe 100. Here, weight of the acoustic probe 100 is an actual weight felt by a user during usage of the acoustic probe 100. In other words, weight of the acoustic probe 100 may include weights of the housing 110 of the acoustic probe 100, the components inside the housing 110, the needle 300, the signal line 330, and the location sensor 140 that are connected to the housing 110, and a portion of the cable 280 connected to the acoustic probe 100.

At least one from between the location calculating unit 150 and the magnetic field control unit 160 may be arranged inside the housing 110 of the acoustic probe 100 or inside the first connector 290 connected to the main body 200. In this case, since at least one from between the location calculating unit 150 and the magnetic field control unit 160 is not arranged inside the main body 200, data processing of the main body 200 may be simplified. Furthermore, if at least one from between the location calculating unit 150 and the magnetic field control unit 160 is arranged at a first connector 290, the acoustic probe 100 gains no additional weight and data processing of the main body 200 may be simplified.

Furthermore, a result detected by the location sensor 140 is transmitted to the acoustic probe 100 via the signal line 330, and the location calculating unit 150 calculates a location of the needle 300 with respect to the housing 110 of the acoustic probe 100 and transmits the location to the main body 200 via cables 280. If the needle 300 is connected to the acoustic probe 100, the main body 200 may receive the calculated location and acoustic signals from the acoustic probe 100 via the single cable 280. Therefore, the number of the cables 280 connected to the main body 200 may be reduced.

Meanwhile, the main body 200 may include an acoustic wave control unit 210 for controlling generation of acoustic waves, an acoustic signal processing unit 220 for generating an acoustic image by using acoustic echo signals, a user input unit 230 for receiving user instructions for generating an acoustic image, and a control unit 250 which controls the components of the acoustic diagnostic system 10, e.g., the magnetic field control unit 160 and the acoustic wave control unit 210, and controls a display unit 240 to display an acoustic image showing location of the needle 300 on the display unit 240.

The components of the acoustic diagnostic system 10 shown in FIG. 2 are only for convenience of explanation and are not essential components of the acoustic diagnostic system 10. The acoustic diagnostic system 10 may be embodied of more components than the components shown in FIG. 2 or less components than the components shown in FIG. 2.

Furthermore, the acoustic wave control unit 210, the acoustic signal processing unit 220, the user input unit 230, and the control unit 250 included in the main body 200 may not be necessarily separated from the acoustic probe 100. At least one from among the components included in the main body 200 may be a component constituting the acoustic probe 100. For example, the acoustic wave control unit 210 or the user input unit 230 may partially be a component of the acoustic probe 100.

The acoustic signal processing unit 220 generates an acoustic image by using acoustic echo signals. An acoustic image may be at least one from among a brightness (B) mode image which indicates intensities of acoustic echo signals reflected by a target object in brightness, a doppler mode image which indicates an image of a moving target object in spectrum using the doppler effect, a motion (M) mode image which indicates movement of a target object at a predetermined location, an elastic mode image which indicates a difference between a reaction when a target object is compressed and a reaction when the target object is not compressed as an image, and a color (C) mode image which indicates velocity of a moving target object in colors by using the doppler effect. Since an acoustic image may be generated by using a method currently known in the art, detailed descriptions thereof will be omitted. Therefore, acoustic images according to an embodiment of the present invention may include all dimensional images, such as 1D images, 2D images, 3D images, and 4D images.

The user input unit 230 generates input data for a user to control operation of the acoustic diagnostic system 10. The user input unit 230 may include a key pad, a dome switch, a touch pad (resistive/capacitive), a jog wheel, a jog switch, etc. Particularly, if a touch pad and the display unit 240 described below constitute a mutual layer structure, the structure may be referred to as a touch screen.

The display unit 240 displays information processed by the acoustic diagnostic system 10. For example, the display unit 240 may display a relative location of the needle 300 in an acoustic image.

The display unit 240 may also display location of the needle 300 inserted into an actual target object. According to an embodiment of the present invention, the display unit 240 may display a path and/or a current location of the needle 300 inserted into a target object.

Meanwhile, as described above, if the display unit 240 and a touch pad constitute a mutual layer structure and are configured as a touch screen, the display unit 240 may be used not only as an output device, but also as an input device. The display unit 240 may include at least one from among a liquid crystal display, a thin-film transistor liquid crystal display, an organic light-emitting diode display, a flexible display, and a 3D display. Furthermore, according to configurations of the acoustic diagnostic system 10, two or more display units 240 may exist.

A touch screen may be configured to detect not only a touch input location and a touched area, but also a touch inputting pressure. Furthermore, a touch screen may be configured to detect not only the real touch, but also a proximity touch.

Here, the term "real touch" refers to a case in which a pointer actually touches a touch screen, whereas the term "proximity touch" refers to a case in which the pointer does not actually touch the touch screen and approaches to a location at a predetermined distance apart from the touch screen. The term "pointer" refers to a tool for touching or proximity-touching a particular point on a displayed screen image. Examples thereof include a stylus pen, a finger, etc. Although not shown, various types of sensors may be arranged inside or nearby the touch screen to detect a touch or a proximity touch on the touch screen.

Meanwhile, since an acoustic image is basically a tomographic image, it is difficult to figure out a relative location relationship between a target object and the needle 300 from an acoustic image. Therefore, the location calculating unit 150 calculates a relative location of the needle 300 with respect to the acoustic probe 100 by using a magnetic field. Next, the control unit 250 may receive the relative location of the needle 300 with respect to the acoustic probe 100 from the location calculating unit 150 and display a needle icon at a location in an acoustic image corresponding to actual location of the needle 300. In this case, relative location of the needle 300 with respect to the acoustic probe 100 may be displayed by a position of the needle icon.

As described above, since a magnetic field generator is included in an acoustic probe, a location relationship between an acoustic image and a needle may be easily figured out.

While the present invention has been particularly shown and described with reference to exemplary embodiments thereof, it will be understood by those of ordinary skill in the art that various changes in form and details may be made therein without departing from the spirit and scope of the present invention as defined by the following claims.

## Claims

1. An acoustic probe comprising:
a housing;
a transducer, which is arranged inside the housing, transmits an acoustic wave to a target object, and receives an acoustic echo signal from the target object; and
a magnetic field generator, which is arranged inside the housing and generates a magnetic field to the target object.

2. The acoustic probe of claim 1, wherein a space in which the magnetic field is formed comprises a direction in which an acoustic wave propagates.

3. The acoustic probe of any preceding claim, wherein a direction of the magnetic field generated by the magnetic field generator is identical to a direction in which an acoustic wave generated by the transducer propagates.

4. The acoustic probe of any preceding claim, wherein the magnetic field generator is formed of a coil.

5. The acoustic probe of any preceding claim, wherein weight of the acoustic probe is less than or equal to about 3Kg.

6. The acoustic probe of any preceding claim, further comprising:
a needle to be inserted into the target object; and
a location sensor, which is arranged at the needle for detecting location of the needle.

7. The acoustic probe of claim 6, wherein the location sensor detects a relative location of location sensor with respect to the magnetic field generator.

8. The acoustic probe of claims 6 or 7, further comprising a location calculating unit, which calculates a relative location of the location sensor with respect to the magnetic field generator based on signals output by the magnetic field generator and/or the location sensor and calculates a relative location of the needle with repsect to the magnetic field generator based on the relative location of the location sensor with respect to the magnetic field generator.

9. The acoustic probe of any one of claims 6 to 8, further comprising a signal line, which interconnects the needle and the housing and transmits a signal detected by the location sensor to the location calculating unit.

10. The acoustic probe of any one of claims 6 to 9, further comprising a magnetic field control unit, which controls magnetic field generation of the magnetic field generator.

11. The acoustic probe of claim 10, wherein the magnetic field control unit is arranged in the housing or the connector that is connected to the acoustic diagnostic system.

12. The acoustic probe of any one of claims 6 to 10, wherein location of the magnetic field generator is a relative location of the magnetic field generator with respect to the location sensor.

13. An acoustic diagnostic system comprising:
the acoustic probe of claim 1; and
an acoustic signal processing unit, which generates an acoustic image by using the acoustic echo signals transmitted by the acoustic probe.

14. The acoustic diagnostic system of claim 13, further comprising a display unit, which displays a needle icon corresponding to the needle and the acoustic image.

15. The acoustic probe of claims 13 or 14, wherein relative location of the needle with respect to the acoustic probe is displayed by a position of the needle icon.
